# FASCICULE DE BREVET EUROPEEN

(11) **EP 2 413 706 B1**
(45) Date de publication et mention de la délivrance du brevet: **08.02.2017**
(21) Numéro de dépôt: 10717683.6
(22) Date de dépôt: 30.03.2010
(51) Int. Cl.: A23D 7/005, A23D 9/007, A23D 9/02, A61K 8/92, A61K 8/97, A61K 9/06, A61K 9/107, A61K 36/53, A61K 36/63, A61K 36/82, A61K 36/85, A61Q 19/00, C11B 5/00, C11B 9/02

(54) **PROCÉDÉ D'EXTRACTION DE COMPOSÉS NON VOLATILS**
VERFAHREN ZUM EXTRAHIEREN VON NICHT FLÜCHTIGEN VERBINDUNGEN
METHOD FOR EXTRACTING NON-VOLATILE COMPOUNDS

(30) Priorité: 30.03.2009 FR 0951982
(43) Date de publication de la demande: 08.02.2012
(73) Titulaire: Oleos, 34130 Mauguio (FR)
(72) Inventeur: Rossignol-Castera, Anne, 34400 Lunel (FR)
(74) Mandataire: Lavoix
(86) Numéro de dépôt international: PCT/FR2010/050591
(87) Numéro de publication internationale: WO 2010/112760

(56) Documents cités:
- EP-A- 0 398 798
- EP-A- 0 639 336
- FR-A- 2 915 350
- US-A- 3 732 111
- US-A- 5 585 130
- VINATORU M: "An overview of the ultrasonically assisted extraction of bioactive principles from herbs" ULTRASONICS SONOCHEMISTRY, vol. 8, 2001, pages 303-313, XP002550202
- Jinxing Cau ET AL: "Study on Extraction Technology of Strawberry Pigments' and Its Physicochemical Properties", Food and Fermentation Industries, 1 January 2003 (2003-01-01), XP055259568, Retrieved from the Internet: URL:http://en.cnki.com.cn/Article_en/CJFDT OTAL-SPFX200305020.htm [retrieved on 2016-03-18]
- L Chen ET AL: "Absorption, distribution, and elimination of tea polyphenols in rats", Drug metabolism and disposition, 1 January 1997 (1997-01-01), pages 1045-1050, XP055259807, Retrieved from the Internet: URL:http://dmd.aspetjournals.org/content/2 5/9/1045.full.pdf [retrieved on 2016-03-18]

## Description

La présente invention concerne un procédé d'extraction de composés naturels non volatils contenus dans une matière première solide d'origine naturelle à l'aide d'un corps gras naturel, notamment une huile végétale, sans utilisation de solvant organique et sans transformation chimique.

Les extraits naturels notamment les extraits de végétaux sont largement utilisés dans de nombreux domaines notamment l'alimentaire, la cosmétologie, la pharmacie. Ces domaines ont des règlementations très précises concernant la toxicité des extraits obtenus et des attentes concernant leur concentration et leur facilité d'emploi.

De nombreuses méthodes d'extraction de composés naturels issus de matières premières naturelles, notamment de végétaux ont été décrites. Dans la plupart des cas, les solvants utilisés sont polaires (eau, glycérine, alcool..), en moindre part apolaires (hexane, acétone, huile minérale, huile végétale, triglycérides à acides gras saturés). Parmi ces solvants, les huiles végétales ont une place particulière, car d'origine naturelle et pouvant être considérées comme des solvants « actifs » largement utilisées en alimentaire et en cosmétique, en particulier pour leur apport en acides gras essentiels oméga 3 et oméga 6 et/ou pour leur apport en composés insaponifiables dont les phytostérols et la vitamine E anti-oxydante.

Parmi les méthodes d'extraction de composés naturels issus de végétaux, on peut notamment citer des méthodes de macération à froid ou à chaud utilisant un simple phénomène de diffusion statique, parfois assisté d'une pression mécanique comme dans le cas des brevets EP0639336 et US5585130. Dans le cas de l'utilisation d'un corps gras comme solvant d'extraction, la méthode de macération est à la fois peu performante en rendement en molécules extraites et mal ou pas maîtrisée au niveau de l'altération oxydative du corps gras. Elle est en particulier mal adaptée au cas des huiles poly-insaturées sensibles à l'oxydation, d'où la préconisation d'utiliser des huiles saturées ou des triglycérides d'acides gras saturés, en particulier si l'extraction se fait à chaud.

Des méthodes de macération dans un solvant de type huile ont été décrites par exemple dans la demande de brevet WO2008/132127 (FR2915350) présentant un procédé d'extraction de composés naturels d'un végétal dans une huile de poisson, par macération à froid, sous atmosphère inerte, à l'abri de la lumière, sans solvant volatil et à température ambiante. Un tel procédé présente l'inconvénient d'être long, la durée de macération étant de l'ordre d'une vingtaine de jours, ce qui peut se traduire par une altération oxydative de l'huile sans obtenir par ailleurs une concentration importante en molécules extraites.

Le brevet FR2693906 décrit une extraction de composés naturels par une huile minérale ou végétale, par macération à une température comprise entre 20 et 60°C. Les algues peuvent contenir une fraction d'eau ou être lyophilisées, le solvant d'extraction peut être de l'eau et l'atmosphère n'est pas contrôlée. Cependant ce type de procédé demande également d'importants volumes d'huile (1 kg de matière biologique pour 20 litres d'huile) et peut être relativement long, d'où les mêmes limites que dans le cas précédent.

Le brevet US3732111 décrit une extraction d'antioxydants d'une épice avec une huile végétale ou animale par chauffage du mélange huile/épice à une température comprise entre 80 à 180°C, le temps d'extraction étant compris entre 30min à environ 3 heures.

Le brevet FR2883003 décrit l'extraction d'antioxydants dans un solvant huileux par macération à chaud. Le traitement thermique étant réalisé par chauffage diélectrique sous fréquence micro-ondes. Ce document préconise de travailler entre 0 et 100 °C et la durée du traitement est de préférence comprise entre 15 min et 14 heures. Les auteurs suggèrent de travailler avec une atmosphère dépourvue d'oxygène, sous pression atmosphérique ou réduite.

Des méthodes d'extraction, assistées par micro-ondes, de composés naturels issus de végétaux ont été décrites. Ces procédés regroupent les extractions faisant intervenir un solvant organique polaire (eau, alcool, glycérine) ou apolaire (hexane). Ainsi, le brevet EP0398798 décrit l'extraction de produits naturels combinant irradiation micro-ondes et solvant organique (hexane, alcool...) sans contrôle de l'atmosphère. Cependant, de tels procédés consomment de grandes quantités de solvants. Ce document préconise de travailler avec un taux d'humidité important (d'environ 40% à 90%) afin de limiter la durée d'irradiation micro-ondes (environ 10 à 100 secondes). Cependant, l'utilisation d'un solvant organique est contraignante pour l'utilisation des extraits ainsi obtenus dans le domaine alimentaire, pharmaceutique ou cosmétique.

Le brevet FR2694300 décrit également un procédé d'extraction dans un solvant huileux non aqueux sous rayonnement micro-ondes. Le rayonnement micro-onde est produit de façon à chauffer principalement les parties aqueuses des plantes plutôt que les composants du substrat. Le temps de chauffe est de l'ordre de 4 à 6 minutes et le traitement est réalisé en présence d'air. Ce procédé est destiné à une aromatisation d'une huile pour des applications alimentaires, sans qu'il soit précisé l'altération oxydative de l'huile et le rendement en molécules extraites.

Des méthodes d'extraction de composés naturels issus de végétaux sous activation ultrasonique ont été également décrites. Le solvant utilisé est en général un solvant polaire (eau, glycérine, alcool) plus rarement apolaire ou huileux. Le traitement est réalisé en présence d'air, avec ou sans chauffage. Ainsi, le brevet FR2803488 concerne l'extraction d'arômes naturels de truffes fraîches à une température de 12°C pendant 3 heures. L'atmosphère n'est pas contrôlée dans ce procédé qui est destiné à une aromatisation d'une huile pour des applications alimentaires.

Un corps gras voit son pouvoir de solubilisation augmenter lorsqu'on le chauffe. Les corps gras, dont les huiles végétales, présentent toutefois l'inconvénient d'être sensibles à l'oxydation, processus accru par la chaleur. Ceci est dû principalement à la présence de doubles liaisons sur leurs acides gras constitutifs, qui ont tendance à s'oxyder. Ceci limite leurs usages ou limite les rendements d'extraction, les utilisateurs étant réticents à utiliser des températures relativement élevées.

La présente invention a pour objectif de remédier aux inconvénients des méthodes d'extraction antérieures en proposant une technique d'extraction à haut rendement employant un corps gras naturel, notamment une huile végétale, dans des conditions combinant avantageusement rapport d'extraction élevé et limitation maîtrisée de l'oxydation de l'huile.

Un autre objectif est de proposer une technique permettant de préserver l'actif extrait, y compris s'il s'agit d'un actif oxydable et/ou photosensible.

Un autre objectif encore de l'invention est de proposer une technique qui permet de conserver les propriétés bénéfiques de l'huile végétale d'extraction, en particulier ses acides gras poly-insaturés natifs et ses composés insaponifiables.

L'inventeur a trouvé qu'il était possible de répondre à tous ces objectifs en passant outre la sensibilité à l'oxydation d'un corps gras naturel, notamment d'une huile végétale et de certains actifs oxydables, et les problèmes de dégradation thermo-oxydative de cette huile en travaillant sur une matière première dispersible et exclusivement sous atmosphère dépourvue ou essentiellement dépourvue d'oxygène et en réalisant un chauffage élevé de très courte durée combiné à une microdispersion de la matière première et rupture des cellules dans le cas où le procédé est appliqué aux végétaux.

La présente invention a ainsi pour objet un procédé d'extraction de composés naturels non volatils, contenus dans une matière première solide d'origine naturelle, notamment une plante, se présentant sous une forme dispersible, à l'aide d'un corps gras naturel ou un mélange de corps gras naturels, notamment une huile végétale ou un mélange d'huiles végétales, caractérisé en ce qu'il comprend :
- au moins une étape a) de mélange et d'imprégnation de la matière première solide avec le corps gras naturel à une température supérieure au point de fusion du corps gras et sous atmosphère dépourvue ou essentiellement dépourvue d'oxygène,
- au moins une étape b) de chauffage à une température comprise entre 80 et 200°C pendant une durée inférieure ou égale à 10 min au moyen de micro-ondes et sous atmosphère dépourvue ou essentiellement dépourvue d'oxygène,
- au moins une étape c) de microdispersion de la matière à extraire avec éventuellement rupture des cellules de la matière première, dans le corps gras naturel à une température supérieure au point de fusion du corps gras et sous atmosphère dépourvue ou essentiellement dépourvue d'oxygène, par un traitement par cavitation ultrasonore,
l'étape c) pouvant être réalisée avant, pendant ou après l'étape b).

Par dispersible, on entend que la matière première se présente sous une forme dissociée apte à être dispersée finement, et par exemple, la matière première est sous forme particulaire et de préférence pulvérulente.

Selon une caractéristique essentielle de l'invention, les étapes unitaires a) b) c) sont conduites en atmosphère dépourvue ou essentiellement dépourvue d'oxygène. Ceci signifie que l'on travaille sous gaz ou atmosphère inerte ou sous vide ou vide partiel. La teneur résiduelle en oxygène doit être suffisamment basse pour ne pas provoquer de réactions d'oxydation sensibles à la température du traitement thermique. On peut donc conduire ces étapes sous atmosphère inerte, par exemple sous azote et de préférence sous balayage continu d'azote, permettant l'extraction de l'oxygène présent ou susceptible de se former. On peut employer un réacteur clos avec une extraction continue de l'oxygène par flux d'azote. On peut aussi faire un barbotage d'azote, associé au flux d'azote, au moins au début du traitement thermique. On peut aussi conduire ces étapes sous vide. Procéder ainsi confère un avantage supplémentaire, à savoir l'entraînement des matières volatiles avec un effet de désodorisation du mélange.

Le procédé selon l'invention est avantageusement réalisé sur un mélange corps gras naturels + matière première contenant de 2 à 40 % d'eau en poids, notamment entre 3 et 30 %, de préférence entre 5 et 15 %. Une partie de l'eau peut notamment provenir du corps gras naturel, le reste étant apporté par la matière première. Un apport d'eau peut être réalisé pour se placer dans ces intervalles.

Parmi les corps gras naturels, on peut employer notamment des huiles végétales raffinées comprenant moins de 0,1 % d'eau en poids ou des huiles végétales vierges ou non raffinées pouvant contenir de 0,1 à 2 % d'eau en poids, de préférence de 0,1 à 0,3 % d'eau en poids. Le Codex Alimentarius fixe la teneur en eau et volatils des huiles végétales alimentaires à 0,2 % en poids.

L'étape a) est conduite à température ambiante ou à une température supérieure au point de fusion du corps gras ou du mélange de corps gras utilisés. Dans un mode de réalisation, la température est avantageusement comprise entre cette température de fusion et la température de fusion + 20° C, de préférence + 10° C. La température ambiante (20-25° C) est parfaitement adaptée pour les corps gras, notamment huiles, liquides à cette température.

La durée de l'étape a) peut être comprise entre 1 et 48 heures, de préférence entre 5 et 30 heures et plus préférentiellement entre 12 et 24 heures, de préférence l'imprégnation est comprise entre 8 et 16 heures, par exemple de l'ordre de 12 heures environ.

Dans un mode de réalisation préférée, la température de l'étape b) est comprise entre 80 et 200° C, de préférence entre 100 et 190°C et plus préférentiellement entre 140 et 170° C.

On entend par chauffage à haute température de très courte durée, un traitement réalisé en un temps inférieur ou égal à 10 minutes, de préférence inférieur ou égale à 5 minutes, de préférence de 1 à 5 minutes, et plus préférentiellement de 1 à 3 minutes, cette durée correspond au temps de maintien de la température de traitement une fois cette température atteinte. Le temps de montée en température est également très court, notamment inférieur ou égal à 5 minutes, de préférence de 1 à 5 min, et plus préférentiellement de 1 à 3 minutes.

Tout système de chauffage thermique rapide peut être utilisé, dans un mode de réalisation préféré, le traitement thermique est assuré par des micro-ondes.

L'utilisation d'une source micro ondes dans un réacteur fermé permet d'atteindre en un temps court les températures souhaitées et ainsi limiter des phénomènes d'oxydation secondaire, d'autant plus que le procédé est mené en atmosphère dépourvue ou essentiellement dépourvue d'oxygène.

Le chauffage à haute température permet d'accroître le pouvoir de solubilisation de l'huile et favorise le contact entre le solvant et le produit à extraire ainsi que la diffusion des solutés dans le solvant. Ainsi, des molécules amphiphiles ou partiellement polaires peuvent être extraites par l'huile chauffée, parallèlement à la solubilisation de molécules d'eau, le rendement de l'extraction s'en trouve donc élevé.

Il est préconisé l'utilisation d'un générateur micro ondes à forte puissance utile, de préférence de l'ordre de 10 000 Watts à 30 000 Watts par kilogramme de mélange.

L'étape c) permet à la fois la microdispersion de la matière à extraire et sur des tissus cellulaires, la rupture des cellules, ce qui favorise la dispersion des molécules extraites dans le corps gras naturel. Cet effet peut être obtenu par les microondes. Suivant une modalité de l'invention, l'étape c) comprend un traitement par micro-ondes. Suivant une certaine modalité, étapes b) et c) sont réalisées par application de micro-ondes, en une ou plusieurs passes.

On entend par microdispersion des particules ou solutés en suspension dans le corps gras. La taille des particules ou solutés est telle qu'il n'y a pas décantation au cours du temps, elle est notamment comprise entre 0.1 à 10 µm.

L'étape c) peut aussi comprendre ou être constituée de traitement du mélange par cavitation ultrasonore avant, pendant ou après l'étape b).

Le traitement micro-onde permet de rompre les membranes cellulaires permettant une meilleure diffusion des composés végétaux intracellulaires dans l'huile. Le traitement ultrasons quant à lui utilise le phénomène de cavitation qui permet de rompre efficacement les membranes cellulaires, de désagréger les particules solides et de disperser de façon homogène les solutés dans l'huile.

La cavitation et la dispersion sous ondes ultrasonores sont de préférence réalisées dans un réacteur fermé équipé d'un générateur d'ultrasons à basse fréquence de cavitation, notamment inférieure à 100 kHz et de préférence de l'ordre de 20 à 30 kHz.

La durée du traitement sous ultrasons est notamment comprise entre 2 et 30 min, de préférence entre 10 et 20 min.

L'étape c) peut être conduite à température ambiante ou à une température supérieure au point de fusion du corps gras ou du mélange de corps gras utilisés. La température est avantageusement comprise entre cette température de fusion et la température de fusion + 20° C, de préférence + 10° C. La température ambiante (20-25° C) est parfaitement adaptée pour les corps gras, notamment huiles liquides à cette température.

Les étapes b) et c) peuvent être conduites en simultané.

L'inventeur a mis en évidence un effet synergique entre un traitement aux micro-ondes et un traitement aux ultrasons permettant une amélioration du rendement d'extraction en un temps plus court et avec, une quantité réduite de corps gras ce qui favorise la concentration finale en molécules extraites.

Suivant une caractéristique avantageuse, on ajoute lors de l'étape c) ou juste avant, un composé piégeur ou réducteur d'oxygène, un composé permettant de régénérer sous forme réduite les tocophérols de l'huile ainsi que les composés phénoliques extraits dans l'huile par le procédé ou un chélateur de métaux pro-oxydants, ces composés contribuant à améliorer la stabilité oxydative du produit final. Il est possible ainsi d'ajouter de la vitamine C, sous forme d'acide ascorbique pur, de sel tel que l'ascorbate de sodium ou de palmitate d'ascorbyle, de l'acide citrique ou de l'acide lactique sous forme libre ou ester ou des lécithines, ou encore une combinaison de ces composés. Il sera ajouté une quantité individuelle de 0,01 à 1 % en poids dans le mélange, préférentiellement de 0,1 à 0,5 % en poids dans le mélange.

Un mode de réalisation préféré est donc le suivant :
- au moins une étape a) de mélange et d'imprégnation de la matière première solide sous forme dispersible avec le corps gras naturel à une température supérieure au point de fusion du corps gras et sous atmosphère dépourvue ou essentiellement dépourvue d'oxygène,
- au moins une étape b) de chauffage à une température comprise entre 80 et 200°C pendant une durée inférieure ou égale à 10 min et sous atmosphère dépourvue ou essentiellement dépourvue d'oxygène, en utilisant des micro-ondes,
- au moins une étape c) de microdispersion de la matière à extraire et éventuellement de rupture des cellules de la matière première dans le corps gras naturel à une température supérieure au point de fusion du corps gras et sous atmosphère dépourvue ou essentiellement dépourvue d'oxygène, en utilisant des ultra-sons,
l'étape c) pouvant être réalisée avant, pendant ou après l'étape b).

Dans ce mode de réalisation préféré, appliqué à de la matière végétale, la matière première est de préférence broyée à basse température, par exemple entre -20 et - 80° C. Par exemple cette étape peut être réalisée par congélation entre -20 et -30° C et broyage (par exemple à l'aide d'un broyeur à couteaux) ou par broyage en conditions cryogéniques (par exemple entre -70 et -80° C). Cette matière se trouve sous forme pulvérulente.

Suivant une caractéristique avantageuse, on ajoute, comme décrit précédemment, lors de l'étape c) ou juste avant, un composé piégeur ou réducteur d'oxygène, un composé permettant de régénérer sous forme réduite les tocophérols de l'huile ainsi que les composés phénoliques extraits dans l'huile par le procédé ou un chélateur de métaux pro-oxydants, ces composés contribuant à améliorer la stabilité oxydative du produit final. Comme décrit précédemment il est possible d'ajouter de la vitamine C, sous forme d'acide ascorbique pur, de sel tel que l'ascorbate de sodium ou de palmitate d'ascorbyle, de l'acide citrique ou de l'acide lactique sous forme libre ou ester ou des lécithines, ou encore une combinaison de ces composés. Il sera ajouté une quantité individuelle de 0,01 à 1 % en poids dans le mélange, préférentiellement de 0,1 à 0,5 % en poids dans le mélange.

Les étapes a) b) c) sont avantageusement conduites en l'absence de lumière ou de tout rayonnement oxydant tels que les UV pour limiter les risques de photo-oxydation et de dégradation des molécules photosensibles.

Les étapes a) b) c) peuvent être réalisées avec ou sans agitation du mélange et préférentiellement avec agitation.

Selon un mode de réalisation le procédé consiste en une séquence combinée des étapes a) b) et c), l'ordre des étapes b) et c) étant indifférent, chaque étape étant réalisée au minimum une fois chacune.

Selon un autre mode de réalisation et en fonction de la matière à extraire et du rendement en molécules extraites souhaitées, les étapes a) b) et c) sont réalisées plusieurs fois.

Selon un mode de réalisation préféré, les étapes b) et c) sont réalisées au moins une seconde fois ; elles correspondent alors aux étapes bn) et cn), n correspondant au nombre total de répétition du cycle {étape a) + étape b)}, n est au moins égal à 2, de préférence n est égal à 2.

Dans un mode de réalisation, il peut être réalisé, entre chaque étape, ou après la dernière étape, une période de diffusion passive des composés extraits dans l'huile et de refroidissement éventuellement sous agitation douce en système fermé sous atmosphère dépourvue ou essentiellement dépourvue d'oxygène.

La durée de cette étape doit être suffisante pour une bonne diffusion des actifs dans l'huile. Cette durée peut être notamment comprise entre 1 h et 24 h heures, de préférence entre 1 h et 12 h.

Le refroidissement peut être réalisé de toute manière connue en soi, notamment par refroidissement passif ou à l'aide de moyens de refroidissement. Cette étape de refroidissement est avantageusement conduite en atmosphère dépourvue ou essentiellement dépourvue d'oxygène, comme les étapes a) b) et c). Elle est avantageusement conduite en l'absence de lumière ou de tout rayonnement oxydant tels que les UV.

A titre d'exemples, on peut mentionner les architectures de procédé suivantes hors étapes préparatoire de broyage et finition définies plus loin:
- étape a) / étape b) / microdiffusion / étape b) /microdiffusion / étape c)
- étape a) / étape c) / microdiffusion / étape b) / microdiffusion / étape c)
- étape a) / étape b) / étape c) / microdiffusion / étape c) micro/diffusion
- étape a) / étape b) / étape c) / microdiffusion / étape b) / étape c)
- étape a) / étapes b) et c) simultanées / microdiffusion / étapes b) et c) simultanées

Le corps gras utilisé comme solvant d'extraction est un corps gras, préférentiellement d'origine végétale, qui peut être soit du type glycéridique (huile végétale) soit du type non glycéridique (cire naturelle) soit un quelconque mélange d'huile(s) végétale(s) et/ou de cire(s) naturelle(s). Le corps gras naturel glycéridique végétal, constitué majoritairement de triesters de glycérol et de trois acides gras, pourra être une huile végétale, un beurre végétal, une graisse végétale, sans limitation ni d'origine, ni de composition, ni de point de fusion. Il pourra s'agir d'huiles à majorité d'acides gras saturés, à majorité d'acides gras mono-insaturés ou à majorité d'acides gras poly-insaturés ou un mélange quelconque de ces trois familles d'acides gras. Les huiles peuvent être sous forme raffinée, sous forme vierge ou sous forme désodorisée. Les cires naturelles peuvent être de cires végétales ou une cire d'abeille. L'invention s'applique également aux huiles végétales interestérifiées, hydrogénées, fractionnées, aux corps gras d'origine animale, aux triglycérides de synthèse, aux esters gras, aux insaponifiables végétaux, au glycérol ou à un quelconque mélange de ces produits.

La matière première d'origine naturelle peut être tout type de matière première, de préférence elle est choisie parmi les plantes terrestres, notamment les parties végétatives aériennes : feuilles, tiges, fleurs, plantes entières, ou bien les racines, tubercules, graines, fruits, tourteaux, farines et tout co-produit de végétaux... les algues microscopiques et macroscopiques ; les champignons ; les lichens ; les produits de la ruche ; les minéraux, roches, sables.. ou un quelconque mélange de ces composés.

Les composés naturels non volatils extraits peuvent être tous types de composés non volatils notamment des composés lipo-solubles, lipo-extractibles ou lipo-dispersibles. Il peut s'agir de molécules apolaires, polaires ou amphiphiles, ou un mélange quelconque de ces composés pouvant avoir des actions synergiques, notamment des composés phénoliques (du type polyphénols, flavonoides, acides phénoliques, catéchines, diterpènes, flavones, monophénols, flavonols glycosides..), des vitamines A,E,C.. libres ou esters, des tannins, des cires, des acides gras, des huiles essentielles, des acides organiques, des protéines hydrophobes, des pigments (caroténoïdes, xanthophylles, lutéine, zéaxanthine, chlorophylles..), des composés insaponifiables (phytostérols libres et estérifiés, alcools gras, triterpènes, squalène..), des lipides polaires (phospholipides, glycolipides, sphingolipides..) des enzymes et co-enzymes, des oligo-éléments, des minéraux, des sels organiques, etc., ou un mélange quelconque de ces composés.

Selon un mode de réalisation de l'invention, le ratio entre la matière première et l'huile dans le mélange de départ mis en oeuvre dans l'étape a) est compris entre 1:0,5 et 1:10, de préférence entre 1:1 et 1:5 et plus préférentiellement entre 1:1 et 1:3 exprimé en masse / masse d'huile ou masse /volume d'huile. Le procédé selon l'invention permet de réduire le ratio entre la matière première et l'huile.

Selon une caractéristique de l'invention, il est avantageux de partir d'une matière première naturelle broyée à basse température, par exemple entre -20 et -80° C. Par exemple cette étape peut être réalisée par congélation entre -20 et -30° C et broyage (par exemple à l'aide d'un broyeur à couteaux) ou par broyage en conditions cryogéniques (par exemple entre -70 et -80° C). Cette étape permet d'augmenter le rendement d'extraction de façon synergique avec l'action de la chaleur, notamment sous micro ondes, et celui du cassage des cellules, notamment par cavitation ultrasonore, ceci par augmentation de la surface de contact entre l'huile et le produit à extraire.

Selon une autre caractéristique de l'invention, la matière naturelle solide est apportée sous la forme d'un produit dispersible obtenu par détente instantanée sous vide de type flash-détente. Cette étape va permettre de rompre les membranes cellulaires, de désagréger les particules solides et d'assurer la dispersion des particules résultantes.

A titre de finition, le procédé peut comprendre une ou plusieurs étape(s) de clarification de l'huile. On entend par clarification toutes les séparations mécaniques connues de l'Homme du métier. Elles peuvent par exemple être choisies entre la filtration, la décantation, la centrifugation, l'essorage, ou une association de ces techniques. Les étapes de clarification permettent l'obtention d'un produit à la fois substantiellement limpide à l'oeil et exempt de microparticules en suspension.

Le procédé selon l'invention permet l'obtention d'un extrait huileux concentré en actifs pouvant se présenter sous forme de solution huileuse, de microdispersion huileuse, de micro suspension huileuse ou de microémulsion huileuse, forme qui soit stable dans le temps.

Le procédé selon l'invention permet d'obtenir simultanément un corps gras végétal et notamment une huile végétale avec un faible niveau d'oxydation et une forte concentration en molécules actives extraites, ceci grâce à la mise en oeuvre simultanée d'un procédé conduit sous atmosphère dépourvue ou essentiellement dépourvue d'oxygène, notamment un inertage azote, et d'un procédé qui utilise l'action synergique d'un traitement court à haute température, notamment sous micro ondes, et de la microdispersion et éventuellement de la rupture des cellules du produit à extraire, notamment par cavitation ultrasonore.

Le faible niveau d'oxydation de l'huile peut s'évaluer à partir de la mesure de l'indice de peroxyde qui évalue la concentration en hydroperoxydes d'acides gras, dits composés primaires d'oxydation, pouvant se former dans l'huile en présence d'oxygène. Cette mesure est réalisée selon une méthode analytique reconnue et normalisée (NF ISO 3960). La teneur en composés extraits peut se mesurer selon différentes méthodes de dosage qui dépendent de la nature de ces composés, par exemple la teneur en composés phénoliques totaux peut se doser selon la méthode colorimétrique de Folin Ciocalteau. la teneur en minéraux par absorption atomique ou torche à plasma, la teneur en vitamines par CLHP.

Le procédé selon l'invention permet également d'obtenir une huile enrichie en composés anti-oxydants entre autres composés, ce qui lui confère une meilleure stabilité à l'oxydation au cours de son stockage et de ses utilisations. Il est ici mis à profit par l'inventeur l'effet synergique connu entre l'activité anti-oxydante des tocophérols naturels des huiles végétales et celle des composés phénoliques et autres molécules anti-oxydantes plus ou moins polaires extraites du végétal ou de la matière première naturelle. Dans le cas de l'extraction de composés réducteurs anti-oxydants et/ou anti-radicalaires, il est possible d'évaluer la capacité anti-oxydante totale de l'extrait huileux obtenu par une méthode in vitro du type test ORAC - Oxygen Radical Absorbance Capacity - qui est largement utilisé et reconnu pour évaluer la capacité d'un extrait végétal à bloquer les réactions radicalaires oxydatives.

Est décrit également un produit sous forme de solution huileuse, micro-dispersion huileuse et microémulsion huileuse susceptible d'être obtenu par la mise en oeuvre du procédé selon l'invention.

Le produit obtenu par l'invention permet d'obtenir des huiles contenant moins de 1% d'eau en poids.

Le procédé selon l'invention permet d'obtenir, à partir d'une matière première naturelle, notamment un végétal, et utilisant un corps gras naturel, notamment une huile végétale, une huile ou corps gras qui présente un état d'oxydation maîtrisé après application du procédé, état pouvant se traduire par un indice de peroxyde inférieur à 10 méqO₂ par kg de produit obtenu, et qui présente simultanément une teneur en composés anti-oxydants phénoliques au minimum 5 à 100 fois supérieure dans le produit obtenu par rapport au corps gras naturel utilisé pris comme référence, le facteur de concentration dépendant directement de la matière première à extraire.

Le produit peut aussi comprendre de l'acide ascorbique libre, sel ou ester, de l'acide citrique ou lactique libre ou ester ou des lécithines.

La présente description décrit également l'utilisation des extraits huileux, solutions huileuses, microémulsions huileuses, micro-suspensions huileuses et micro-dispersions huileuses, d'origine 100% végétale ou naturelle ou biologique, obtenues selon le procédé, dans des formulations, avantageusement des formulations sous forme d'émulsions [phase aqueuse + phase huileuse] ou des formulations 100 % phase huileuse, pour des applications dans le domaine alimentaire, nutritionnel, cosmétique, pharmaceutique ou vétérinaire.

La présente description concerne une formulation alimentaire, nutritionnelle, cosmétique, pharmaceutique ou vétérinaire, contenant un produit selon l'invention.

La présente description concerne une formulation cosmétique notamment de type huile, crème, savon, sérum, lait, maquillage, lotion, shampooing, etc.

L'invention et ses avantages, notamment en termes de rendement d'extraction et d'absence de dégradation oxydative de l'huile, sera mieux comprise à la lecture des exemples suivants qui doivent être considérés à titre non limitatif et qui concernent l'application du procédé à l'extraction de composés phénoliques à partir de différentes plantes.

### Exemple 1

100 g de feuilles d'olivier entières et séchées à l'air sont broyées à -80°C à l'aide d'un broyeur à couteaux pendant 1 min afin d'obtenir une poudre fine et homogène puis la poudre obtenue est mélangée avec 500 ml d'huile de tournesol raffinée. L'étape a) est conduite en système fermé pendant 2 h après bullage d'azote et à température ambiante. L'étape b) est conduite sous balayage azote et sous une puissance de micro ondes de 800 watts en 2 x 3 min. La température maximale atteinte est de 145 °C. L'étape c) est conduite sous balayage d'azote et sous une fréquence d'ultrasons de 20 kHz pendant 2 x 3 min. L'huile est séparée par centrifugation à 5000 tours / min pendant 5 min puis filtrée. La teneur en composés phénoliques totaux dosés par la méthode de Folin Ciocalteau en équivalent oléuropéine, passe de < 20 ppm (mg / kg) pour l'huile de tournesol seule à 1038 ppm (mg/kg) pour l'extrait huileux obtenu selon le procédé. A titre de comparaison, le même dosage des composés phénoliques totaux réalisé sur un produit commercial obtenu par simple macération de feuilles d'olivier dans une huile donne une valeur de 26 ppm (mg / kg). L'indice de peroxyde de l'huile de tournesol avant extraction est de 2,2 ± 0,5 méq O₂/kg et celui de l'extrait huileux obtenu selon le procédé est de 3,3 ± 0,5 méq O₂/kg. Après une semaine à température ambiante, à l'abri de l'air et de la lumière, l'indice de peroxyde de l'extrait huileux obtenu selon le procédé reste inférieur à 4 ± 1 méq O₂/kg.

### Exemple 2

Identique à l'exemple 1 mais avec l'huile de pépin de raisin raffinée. La teneur en composés phénoliques totaux dosés par la méthode de Folin Ciocalteau en équivalent oléuropéine passe de < 20 ppm (mg / kg) pour l'huile de pépin de raisin seule à 826 ppm (mg/kg) pour l'extrait huileux obtenu selon le procédé. La capacité anti-radicalaire ORAC de l'huile de pépin de raisin est de 300 micromoles de Trolox équivalent / kg et elle passe, pour l'extrait huileux obtenu selon le procédé, à 7860 micromoles de Trolox équivalent / kg (soit 26 fois plus).

### Exemple 3

150 g de feuilles de romarin séchées sont broyées à -25°C à l'aide d'un broyeur à couteaux et mélangées avec 450 ml d'huile de tournesol raffinée. L'étape a) est conduite en système fermé pendant 12 h après bullage d'azote et saturation de l'espace de tête en azote et à température ambiante. L'étape b) est conduite en réacteur sous balayage continu d'azote, sous agitation et sous une puissance de micro ondes de 1200 watts en 2 x 4 min. La température maximale atteinte est de 152 °C. L'étape c) est conduite en réacteur sous balayage d'azote, sous agitation et sous une fréquence d'ultrasons de 20 kHz pendant 20 min. L'huile est séparée par essorage centrifuge. La teneur en composés phénoliques totaux dosés par la méthode de Folin Ciocalteau en équivalent oléuropéine passe de < 20 ppm (mg / kg) pour l'huile de pépin de raisin seule à 1535 ppm (mg/kg) pour l'extrait huileux obtenu selon le procédé. L'indice de peroxyde de l'extrait huileux obtenu selon le procédé reste inférieur à 5 ± 1 méq O₂/kg après deux semaines de conservation à température ambiante, à l'abri de l'air et de la lumière.

### Exemple 4

Pour un extrait huileux obtenu selon le procédé décrit à l'exemple 3 ayant une teneur en composés phénoliques totaux dosés par la méthode de Folin Ciocalteau en équivalent oléuropéine de 2350 ppm (mg / kg), on obtient une capacité anti-radicalaire ORAC de 13 100 micromoles de Trolox équivalent / kg, soit 65 fois plus que l'huile de pépin de raisin raffinée qui a une valeur ORAC mesurée avec le même protocole analytique de 200 micromoles de Trolox équivalent / kg. Ce même produit obtenu selon le procédé décrit à l'exemple 3 garde un indice de peroxyde inférieur à 6 ± 1 méq O₂/kg après 6 mois de conservation au frigo, à l'abri de l'air et de la lumière.

### Exemple 5

100 g de feuilles de romarin séchées sont broyées à - 25°C à l'aide d'un broyeur à couteaux et mélangées avec 300 g d'huile d'olive vierge. L'étape a) est conduite en système fermé pendant 12 h après bullage d'azote et à température ambiante. L'étape c) est conduite en réacteur avec double enveloppe, à une température de 40°C et sous une fréquence d'ultrasons de 25 kHz. L'étape c) est conduite pendant 20 min sous balayage d'azote et sous agitation douce. L'étape b) est conduite en réacteur sous balayage azote, sous agitation et sous une puissance de micro-ondes de 1200 watts pendant 3 min. La température maximale atteinte est de 145 °C. L'étape c) est à nouveau conduite en réacteur sous balayage d'azote, sous agitation et sous une fréquence d'ultrasons de 25 kHz, pendant 10 min. L'huile est séparée par essorage centrifuge. La teneur en composés phénoliques totaux dosés par la méthode de Folin Ciocalteau en équivalent oléuropéine est de 1305 ppm (mg/kg) pour l'extrait huileux obtenu selon le procédé avec parallèlement un indice de peroxyde de 4 ± 1 méq O₂/kg.

### Exemple 6

Formulations d'un extrait huileux obtenu selon le procédé dans une crème cosmétique. Un extrait huileux est préparé selon l'exemple 2 à partir de feuilles d'olivier et d'huile de pépin de raisin raffinée. Cet extrait huileux a été formulé dans une crème cosmétique de base contenant une phase aqueuse A et une phase grasse B, comme décrit dans les tableaux 1 & 2. L'extrait huileux a été incorporé à une dose de 0,5 % en poids soit dans la phase B (formulation 1, tableau 1), soit en fin de formulation (formulation 2, tableau 2). Les crèmes 1 et 2 ont été préparées selon le mode opératoire suivant : pesée de la phase A dans un bécher en inox, pesée de la phase B dans un autre bécher en inox, chauffage des deux phases à 75°C, agitation de la phase A à 400 tours/min avec un agitateur mécanique IKA modèle EURO-ST P CV, incorporation de la phase B, agitation pendant dix minutes à température ambiante, passage deux minutes à l'ultra- turrax modèle IKAT 25V à 9000 tours/min, refroidissement dix minutes avec l'agitateur mécanique à 400 tours/min dans un bain d'eau froide, incorporation sous agitation de la phase C puis de la phase D. Le pH de l'émulsion finale est de 5,7.

**Tableau 1 - Formulation 1**

| **Phase** | **Ingrédient** | **Fournisseur** | **%** |
|---|---|---|---|
| **A** | Eau déminéralisée | - | **78** |
| | Glycérine | Cooper | **3** |
| **B** | Montanov 202 | Seppic | **3** |
| | Simulsol 165 | Seppic | **2** |
| | Huile de paraffine | Shell | **5** |
| | Huile de tournesol | Cauvin | **5** |
| | Tegosoft TN | Evonik | **2** |
| | **Extrait huileux** | - | **0,5** |
| **C** | Sepigel 305 | Seppic | **1** |
| **D** | Sepicide HB | Seppic | **0,5** |

**Tableau 2 - Formulation 2**

| **Phase** | **Ingrédient** | **Fournisseur** | **%** |
|---|---|---|---|
| **A** | Eau déminéralisée | - | **78** |
| | Glycérine | Cooper | **3** |
| **B** | Montanov 202 | Seppic | **3** |
| | Simulsol 165 | Seppic | **2** |
| | Huile de paraffine | Shell | **5** |
| | Huile de tournesol | Cauvin | **5** |
| | Tegosoft TN | Evonik | **2** |
| **C** | Sepigel 305 | Seppic | **1** |
| **D** | **Extrait huileux** | - | **0,5** |
| | Sepicide HB | Seppic | **0,5** |

L'analyse microscopique révèle pour chaque formulation 1 et 2, une émulsion huile dans eau, fine et homogène, avec des globules gras de taille inférieure au micron. Les deux formulations 1 et 2 sont stables après 28 jours en étuve à température ambiante et à 40°C et ne conduisent pas à un déphasage (tableau 3).

**Tableau 3 - Tests de stabilité des formulations 1 & 2**

| | **1 semaine** | | **2 semaines** | | **3 semaines** | | **4 semaines** | |
|---|---|---|---|---|---|---|---|---|
| | **ambiante** | **40°C** | **ambiante** | **40°C** | **ambiante** | **40°C** | **ambiante** | **40°C** |
| Crème de base | Stable | Stable | Stable | Stable | Stable | Stable | Stable | Stable |
| Formulation 1 avec 0,5 % d'extrait huileux obtenu selon le procédé ajouté dans la phase B | Stable | Stable | Stable | Stable | Stable | Stable | Stable | Stable |
| Formulation 1 avec 0,5 % d'extrait huileux obtenu selon le procédé ajouté en fin de formulation | Stable | Stable | Stable | Stable | Stable | Stable | Stable | Stable |

## Revendications

1. Procédé d'extraction de composés naturels non volatils, contenus dans une matière première solide d'origine naturelle, notamment une plante, sous une forme dispersible, à l'aide d'un corps gras naturel ou un mélange de corps gras naturels, notamment une huile végétale ou un mélange d'huiles végétales, **caractérisé en ce qu'**il comprend :
- au moins une étape a) de mélange et d'imprégnation de la matière première solide sous forme dispersible avec le corps gras naturel à une température supérieure au point de fusion du corps gras et sous atmosphère dépourvue ou essentiellement dépourvue d'oxygène,
- au moins une étape b) de chauffage à une température comprise entre 80 et 200°C pendant une durée inférieure ou égale à 10 minutes au moyen de micro-ondes et sous atmosphère dépourvue ou essentiellement dépourvue d'oxygène,
- au moins une étape c) de microdispersion de la matière solide et éventuellement de rupture des cellules de la matière première, dans le corps gras naturel à une température supérieure au point de fusion du corps gras et sous atmosphère dépourvue ou essentiellement dépourvue d'oxygène, par un traitement par cavitation ultrasonore,
l'étape c) pouvant être réalisée avant pendant ou après l'étape b).

2. Procédé selon la revendication 1, **caractérisé en ce que** le mélange corps gras naturel + matière première contient entre 2 et 40% d'eau en poids.

3. Procédé selon l'une quelconque des revendications précédentes, **caractérisé en ce que** l'étape a) est conduite à une température comprise entre la température de fusion et la température de fusion + 20°C,

4. Procédé selon l'une quelconque des revendications précédentes, **caractérisé en ce que** la durée de l'étape a) est comprise entre 1 et 48 heures,

5. Procédé selon l'une quelconque des revendications précédentes, **caractérisé en ce que** le ratio entre la matière première et l'huile dans l'étape a) est compris entre 1:0,5 et 1:10, ratio exprimé en masse/masse d'huile ou en masse/volume d'huile.

6. Procédé selon l'une quelconque des revendications 1 à 5, **caractérisé en ce que** l'on emploie des micro-ondes de forte puissance utile comprise entre 10 000 et 30 000 Watts par kg de mélange à traiter.

7. Procédé selon l'une quelconque des revendications précédentes, **caractérisé en ce que** la température de l'étape b) est comprise entre 100 et 190°C

8. Procédé selon l'une quelconque des revendications précédentes, **caractérisé en ce que** la durée de l'étape b) est inférieure ou égale à 5 minutes.

9. Procédé selon l'une quelconque des revendications précédentes, **caractérisé en ce que** l'étape c) est mise en oeuvre en présence d'acide ascorbique libre, sel ou ester, d'acide citrique ou lactique libre ou ester, ou de lécithines.

10. Procédé selon l'une quelconque des revendications précédentes, **caractérisé en ce que** la durée du traitement ultrasons est comprise entre 2 et 30 minutes, et/ou en ce que la fréquence de cavitation est inférieure à 100 kHz.

11. Procédé selon l'une quelconque des revendications précédentes, **caractérisé en ce que** la matière première solide est apportée sous la forme d'un produit dispersible obtenu par broyage à une température comprise entre -20 et -80° C et/ou sous la forme d'un produit dispersible obtenu par détente instantanée sous vide de type flash-détente.

12. Procédé selon l'une quelconque des revendications précédentes, **caractérisé en ce qu'**il est conduit en l'absence de lumière ou de tout rayonnement oxydant tel que les UV ou **en ce qu'**il est réalisé entre chaque étape ou après la dernière étape, une période de diffusion passive des extraits dans l'huile et de refroidissement sous atmosphère dépourvue ou essentiellement dépourvue d'oxygène et/ou **en ce que** les étapes a), b) et c) sont réalisées avec agitation et/ou **en ce que** l'une, plusieurs ou la totalité des étapes a), b) et c) sont réalisées en plusieurs fois.

13. Procédé selon l'une quelconque des revendications précédentes **caractérisé en ce que** le corps gras utilisé comme solvant d'extraction est un corps gras, choisi parmi les corps gras du type glycéridique; les corps gras du type non glycéridique; ou un mélange et/ou **en ce que** la matière première d'origine naturelle est choisie parmi les plantes terrestres, et parmi les parties végétatives aériennes : feuilles, tiges, fleurs, plantes entières, ou bien les racines, tubercules, graines, fruits, tourteaux, farines et tout co-produit de végétaux ; les algues microscopiques et macroscopiques ; les champignons ; les lichens ; les produits de la ruche ; les minéraux, roches, sables, ou un quelconque mélange de ces composés et/ou **en ce que** les composés naturels non volatils extraits sont tous types de composés non volatils choisis parmi des composés lipo-solubles, lipo-extractibles ou lipo-dispersibles.

14. Procédé selon l'une quelconque des revendications précédentes **caractérisé en ce que** les composés non volatils naturels sont des molécules apolaires, polaires ou amphiphiles choisi parmi les composés phénoliques, du type polyphénols, flavonoïdes, acides phénoliques, catéchines, diterpènes, flavones, monophénols, flavonols glycosides ; des vitamines de type vitamines A,E,C libres ou esters ; des tannins ; des cires ; des acides gras ; des huiles essentielles ; des acides organiques ; des protéines hydrophobes ; des pigments; des composés insaponifiables; des lipides polaires; des enzymes et co-enzymes ; des oligo-éléments ; des minéraux ; des sels organiques ou un mélange quelconque de ces composés.

## Patentansprüche

1. Verfahren zur Extraktion von nichtflüchtigen natürlichen Verbindungen, die in einem ersten Feststoff natürlichen Ursprungs, insbesondere einer Pflanze, in dispergierbarer Form enthalten sind, mittels eines natürlichen Fetts oder einer Mischung von natürlichen Fetten, insbesondere eines Pflanzenöls oder einer Mischung von Pflanzenölen, **dadurch gekennzeichnet, dass** es umfasst:
- mindestens einen Schritt a) der Mischung und Imprägnierung des ersten Feststoffs in dispergierbarer Form mit dem natürlichen Fett bei einer Temperatur höher als der Schmelzpunkt des Fetts und unter einer Atmosphäre, die keinen oder im Wesentlichen keinen Sauerstoff aufweist,
- mindestens einen Schritt b) der Aufheizung auf eine Temperatur zwischen 80 und 200°C während einer Dauer kleiner oder gleich 10 Minuten mit Hilfe von Mikrowellen und unter einer Atmosphäre, die kein oder im Wesentlichen kein Sauerstoff aufweist,
- mindestens einen Schritt c) der Mikrodispersion des Feststoffs und gegebenenfalls des Aufbrechens der Zellen des ersten Stoffs in dem natürlichen Fett bei einer Temperatur über dem Schmelzpunkt des Fetts und unter einer Atmosphäre, die kein oder im Wesentlichen kein Sauerstoff aufweist, durch Ultraschallkavitation,
wobei der Schritt c) vor, während oder nach dem Schritt b) realisiert werden kann.

2. Verfahren nach Anspruch 1, **dadurch gekennzeichnet, dass** die Mischung des natürlichen Fetts und des ersten Stoffs zwischen 2 und 40 Gew.-% Wasser enthält.

3. Verfahren nach einem beliebigen der vorhergehenden Ansprüche, **dadurch gekennzeichnet, dass** der Schritt a) bei einer Temperatur zwischen der Schmelztemperatur und der Schmelztemperatur plus 20°C durchgeführt wird.

4. Verfahren nach einem beliebigen der vorhergehenden Ansprüche, **dadurch gekennzeichnet, dass** die Dauer des Schritts a) zwischen 1 und 48 Stunden liegt.

5. Verfahren nach einem beliebigen der vorhergehenden Ansprüche, **dadurch gekennzeichnet, dass** das Verhältnis zwischen dem ersten Stoff und dem Öl im Schritt a) zwischen 1:0,5 und 1:10 liegt, wobei das Verhältnis ausgedrückt ist in Masse/Masse des Öls oder in Masse/Volumen des Öls.

6. Verfahren nach einem beliebigen der Ansprüche 1 bis 5, **dadurch gekennzeichnet, dass** Mikrowellen mit einer hohen Leistungsabgabe zwischen 10 000 und 30 000 Watt pro kg der zu behandelnden Mischung verwendet werden.

7. Verfahren nach einem beliebigen der vorhergehenden Ansprüche, **dadurch gekennzeichnet, dass** die Temperatur des Schritts b) zwischen 100 und 190°C liegt.

8. Verfahren nach einem beliebigen der vorhergehenden Ansprüche, **dadurch gekennzeichnet, dass** die Dauer des Schritts b) kleiner oder gleich 5 Minuten ist.

9. Verfahren nach einem beliebigen der vorhergehenden Ansprüche, **dadurch gekennzeichnet, dass** der Schritt c) in Anwesenheit von freier Ascorbinsäure, dessen Salz oder dessen Ester, freier Zitronensäure oder freier Milchsäure oder deren Ester, oder Lecithinen durchgeführt wird.

10. Verfahren nach einem beliebigen der vorhergehenden Ansprüche, **dadurch gekennzeichnet, dass** die Dauer der Ultraschallbehandlung zwischen 2 und 30 Minuten liegt, und/oder dass die Kavitationsfrequenz kleiner als 100 kHz ist.

11. Verfahren nach einem beliebigen der vorhergehenden Ansprüche, **dadurch gekennzeichnet, dass** der erste Feststoff in der Form eines dispergierbaren Produkts beschafft wird, das durch Zerkleinern bei einer Temperatur zwischen -20 und -80°C erhalten wird und/oder in Form eines dispergierbaren Produkts, das durch unverzögerte Entspannung unter Vakuum nach Art einer Blitz-Entspannung erhalten wird.

12. Verfahren nach einem beliebigen der vorhergehenden Ansprüche, **dadurch gekennzeichnet, dass** es unter Ausschluss von Licht oder jeder oxidierenden Strahlung, wie UV-Strahlung, durchgeführt wird, oder dass zwischen jedem Schritt oder nach dem letzten Schritt eine Periode der passiven Diffusion der Extrakte in dem Öl und der Abkühlung unter einer Atmosphäre, die keinen oder im Wesentlichen keinen Sauerstoff aufweist, realisiert wird, und/oder dass die Schritte a), b) und c) unter Rühren durchgeführt werden, und/oder dass der eine, die mehreren oder die Gesamtheit der Schritte a), b) und c) mehrere Male realisiert werden.

13. Verfahren nach einem beliebigen der vorhergehenden Ansprüche, **dadurch gekennzeichnet, dass** das als Extraktionslösungsmittel verwendete Fett ein Fett ist, ausgewählt aus den Fetten des Glyzerin-Typs; den Fetten des Nichtglyzerin-Typs; oder einer Mischung und/oder dass der erste Stoff natürlichen Ursprungs ausgewählt ist aus Bodenpflanzen und aus den oberirdischen Pflanzenteilen: Blättern, Stielen, Blumen, gesamten Pflanzen oder auch Wurzeln, Knollen, Körnern, Früchten, Presskuchen, Mehlen und jedem Pflanzennebenprodukt; mikroskopischen und makroskopischen Algen; Pilzen; Flechten; Bienenstockprodukten; Mineralien, Steinen, Sanden oder eine beliebige Mischung dieser Komponenten und/oder dass die extrahierten nichtflüchtigen natürlichen Verbindungen alle solche Art von nichtflüchtigen Verbindungen sind, die ausgewählt sind aus lipidlöslichen Verbindungen, lipidextrahierbaren Verbindungen oder lipiddispergierbaren Verbindungen.

14. Verfahren nach einem beliebigen der vorhergehenden Ansprüche, **dadurch gekennzeichnet, dass** die nichtflüchtigen natürlichen Verbindungen apolare, polare oder amphiphile Moleküle sind, ausgewählt aus den Phenolverbindungen, wie Polyphenole, Flavonoide, Phenolsäuren, Katechinen, Diterpenen, Flavonen, Monophenolen, Glykosidflavonen; Vitaminen der Art freier Vitamine A, E, C oder deren Ester; Tanninen; Wachsen; Fettsäuren; ätherischen Ölen; organischen Säuren; hydrophoben Proteinen; Pigmenten; unverseifbaren Verbindungen; polaren Lipiden; Enzymen und Koenzymen; Oligoelementen; Mi-neralien; organischen Salzen oder einer beliebigen Mischung dieser Komponenten.

## Claims

1. Method for extracting non-volatile natural compounds contained in a first solid material of natural origin, in particular a plant, in a dispersible form, with the help of a natural fatty substance or a mixture of natural fatty substances, in particular a vegetable oil or a mixture of vegetable oils, **characterised in that** it comprises:
- at least one step a) of mixing and impregnating the first solid material in a dispersible form with the natural fatty substance at a temperature which is greater than the melting point of the fatty substance and under an atmosphere which is depleted or essentially depleted in oxygen,
- at least one step b) of heating to a temperature between 80 and 200°C for a duration which is less than or equal to 10 minutes by means of microwaves and under an atmosphere which is depleted or essentially depleted in oxygen,
- at least one step c) of microdispersing the solid material, and possibly of rupturing the cells of the first material, into the natural fatty substance at a temperature which is greater than the melting point of the fatty substance and under an atmosphere which is depleted or essentially depleted in oxygen, by an ultrasonic cavitation treatment,
step c) being able to be implemented before, during or after step b).

2. Method according to claim 1, **characterised in that** the mixture of natural fatty substance + first material comprises between 2 and 40% by weight of water.

3. Method according to any of the preceding claims, **characterised in that** step a) is implemented at a temperature between the melting temperature and the melting temperature + 20°C.

4. Method according to any of the preceding claims, **characterised in that** the duration of step a) is between 1 and 48 hours.

5. Method according to any of the preceding claims, **characterised in that** the ratio between the first material and the oil in step a) is between 1 : 0.5 and 1 : 10, the ratio expressed in mass/mass of oil or in mass/volume of oil.

6. Method according to any of the claims 1 to 5, **characterised in that** useable high-power microwaves, of between 10,000 and 30,000 watts, are used per kg of the mixture to be treated.

7. Method according to any of the preceding claims, **characterised in that** the temperature in step b) is between 100 and 190°C.

8. Method according to any of the preceding claims, **characterised in that** the duration of step b) is less than or equal to 5 minutes.

9. Method according to any of the preceding claims, **characterised in that** step c) is implemented in the presence of free ascorbic acid, salt or ester, of free citric or lactic acid or ester, or of lecithins.

10. Method according to any of the preceding claims, **characterised in that** the duration of the ultrasonic treatment is between 2 and 30 minutes, and/or **in that** the cavitation frequency is less than 100 kHz.

11. Method according to any of the preceding claims, **characterised in that** the first solid material is provided in the form of a dispersible product which is obtained by grinding at a temperature between - 20 and -80°C and/or in the form of a dispersible product which is obtained by instantaneous release under vacuum of the flash release type.

12. Method according to any of the preceding claims, **characterised in that** it is implemented in the absence of light or of any oxidising radiation, such as UV, or **in that** there is implemented between each step or after the last step, a passive diffusion period of the extracts in the oil and of cooling under an atmosphere which is depleted or essentially depleted in oxygen, and/or **in that** steps a), b) and c) are implemented with agitation and/or **in that** one, several or all of steps a), b) and c) are implemented several times.

13. Method according to any of the preceding claims, **characterised in that** the fatty substance which is used as extraction solvent is a fatty compound, chosen from fatty substances of the glyceridic type; fatty substances of the non-glyceridic type; or a mixture and/or **in that** the first material of natural origin is chosen from terrestrial plants, and from aerial vegetative parts: leaves, stalks, flowers, whole plants, or even roots, tubers, grains, fruits, cattlecake, flours and any vegetable co-product; microscopic and macroscopic algae; mushrooms; lichens; bee-keeping products; minerals, rocks, sands or any mixture of these compounds and/or **in that** the natural non-volatile compounds which are extracted are any types of non-volatile compounds chosen from lipo-soluble, lipo-extractable or lipo-dispersible compounds.

14. Method according to any of the preceding claims, **characterised in that** the natural non-volatile compounds are nonpolar, polar or amphiphilic molecules chosen from phenolic compounds, of the polyphenol, flavonoid, phenolic acid, catechin, diterpene, flavone, monophenol, glycoside flavonol type; vitamins of the free A, E, C vitamin type or esters; tannins; waxes; fatty acids; essential oils; organic acids; hydrophobic proteins; pigments; non-saponifiable compounds; polar lipids; enzymes and co-enzymes; oligo-elements; minerals; organic salts or a mixture of any of these compounds.
